(19) Europäisches Patentamt
European Patent Office
Office européen des brevets

(11) EP 2 778 167 A1

(12) EUROPEAN PATENT APPLICATION

(43) Date of publication:
**17.09.2014 Bulletin 2014/38**

(21) Application number: **13001214.9**

(22) Date of filing: **11.03.2013**

(51) Int Cl.:
***C07D 453/02*** *(2006.01)*
***A61K 31/439*** *(2006.01)*
***A61K 9/16*** *(2006.01)*
***A61K 47/08*** *(2006.01)*
***A61K 47/38*** *(2006.01)*
***A61K 47/30*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Abdi Ibrahim Ilac Sanayi ve Ticaret Anonim Sirketi**
**Istanbul (TR)**

(72) Inventor: **Ferhat, Farsi**
**34555 Istambul (TR)**

(54) **Stable pharmaceutical composition comprising amorphous solifenacin or its pharmaceutically acceptable salt**

(57) The present invention relates to a pharmaceutical composition that has improved storage stability with respect to dissolution comprising at least 2% w/w disintegrant(s) and solifenacin or pharmaceutical acceptable salt thereof which is substantially free of crystalline form and is free of degradation impurities, characterized in that the composition is prepared by wet granulation.

EP 2 778 167 A1

Printed by Jouve, 75001 PARIS (FR)

## Description

### Technical Field

The present invention relates to a pharmaceutical composition that has improved storage stability with respect to dissolution comprising at least 2% w/w disintegrant (s) and solifenacin or pharmaceutical acceptable salt thereof which is substantially free of crystalline form and is free of degradation impurities, characterized in that the composition is prepared by wet granulation.

### Background of the Invention

Solifenacin, chemically (3R)-1-azabicyclo[2.2.2]oct-3-yl-(1S)-1-phenyl-3,4-dihydroisoquinoline-2(1H)-carboxylate or (S)-phenyl-1,2,3,4-tetrahydroisoquinoline-2-carboxylic acid 3(R)-quinuclidinyl ester, is a urinary antispasmodic of the antimuscarinic class, useful for the treatment of overactive bladder that was originally disclosed in EP0801067. Solifenacin is commercially available in particular in succinate salt. Solifenacin succinate has the molecular formula $C_{23}H_{26}N_2O_2{\bullet}C_4H_6O_4$, and a molecular weight of 480.55. The structural formula of solifenacin succinate is:

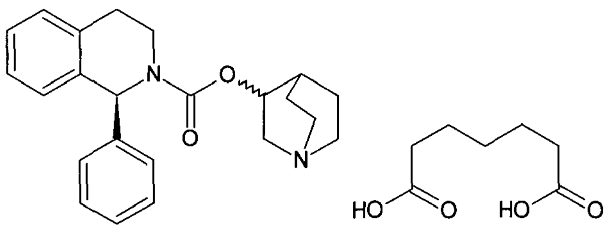

**Formula I**

The commercial solifenacin succinate tablet formulation is marketed under the trade name VESIcare®. Each VESIcare® tablet contains solifenacin succinate as active ingredients and lactose monohydrate, corn starch, hypromellose 2910 and magnesium stearate as inactive excipents.

EP1728791A1 describes a pharmaceutical composition of solifenacin or salt thereof comprising crystalline solifenacin or salt thereof, wherein ratio of amorphous content is not too high. To obtain the pharmaceutical composition in industrial scale is difficult. The application also addresses that direct tabletting is disadvantageous due to strong aggregation properties of solifenacin and its salts leading to poor content uniformity and sticking of the mixture to the punches during compression.

EP2018850 A1 describes amorphous solifenacin composition comprises a stabilizer and the ratio of the stabilizer to amorphous solifenacin or a pharmaceutically acceptable salts thereof based on parts per weight is at least about 2:1. Stabilizers are typical binders to keep solifenacin in amorphous state. The disadvantage of this invention is that the applied spray dry technique is difficult and not feasible to apply due to difficulties to remove residual solvent trapped in spheres.

EP2415472 A1 describes solid pharmaceutical composition, comprising an amorphous form of solifenacin or a pharmaceutically acceptable salt thereof, and one stabilizer for amorphous solifenacin or two or more stabilizers for amorphous solifenacin, selected from the group consisting of citric acid or a pharmaceutically acceptable salt (excluding a calcium salt) thereof, sodium pyrosulfite, and a pharmaceutically acceptable salt of ethylenediaminetetra acetic acid. The above prior disclosed process for the preparation of pharmaceutical composition of solifenacin or a pharmaceutically acceptable salt by freeze drying or spray drying is not industrially feasible and is expensive.

EP2400954A1 describes pharmaceutical compositions comprising solifenacin or its pharmaceutically acceptable salts as the active pharmaceutical ingredient prepared by dry technological methods, preferably by the direct compression or roller compaction method, i.e. by the process in the absence of a liquid solvent, preferable in the absence of water. The same patent application also discloses the preparation of pharmaceutical composition of solifenacin by direct compression which is not preferable during scale up because of technical disadvantages such as sticking, and absence of robustness.

EP1832288B11 describes a pharmaceutical composition comprising solifenacin or a salt thereof, and a binder which stabilizes the solifenacin or salt thereof, and a binder which stabilizes the solifenacin or salt thereof, the binder having a glass transition point or melting point lower than 174 °C; wherein the binder is one or more of polyethylene glycol, polyethylene oxide, a polyoxyethylene/polyoxypropylene block copolymer, hydroxypropyl cellulose, hydroxyethyl cellulose, ethyl cellulose and maltose.

WO 2008/128028A2 describes a process for the preparation of stabilized solifenacin or salt thereof with an antioxidant and complexation agents such as cyclodextrin and resinate. These methods include precipitation which needs additional sieve and drying steps or complexation with cyclodextrin which is expensive or resinate with difficult to remove residual solvent hence not feasible.

**Summary of the Invention**

The object of the present invention is to develop a pharmaceutical composition that has improved storage stability with respect to dissolution comprising at least 2% w/w disintegrant (s) and solifenacin or pharmaceutical acceptable salt thereof which is free of degradation impurities, characterized in that the composition is prepared by wet granulation.

In one aspect, the present invention discloses a pharmaceutical composition comprising Solifenacin or pharmaceutical acceptable salt thereof which is substantially free of crystaline form.

In other aspect, the present invention discloses a pharmaceutical composition comprising solifenacin or pharmaceutical acceptable salt thereof and at least 2% w/w disintegrant (s) shows a dissolution profile with a variability of dissolution from the different tablet samples of a set of below 20%, measured using USP apparatus 2, placing the tablets in 900 ml 0.1 N HCl at 37 ± 0.5 °C with paddle speed of 50 rpm. A significant advantage of the present invention is the dissolution rate shows less variability vis-à-vis commercial comprasion product.

**Detailed Description of the Invention**

In one aspect, the invention relates to a pharmaceutical composition that has improved storage stability with respect to dissolution comprising at least 2% w/w disintegrant(s) and solifenacin or pharmaceutical acceptable salt thereof which is substantially free of crystalline form and it is free of degradation impurities, characterized in that the composition is prepared by wet granulation.

It has been surprisingly found that the pharmaceutical formulations according to the present invention have superior dissolution profiles over the comparison product, especially in terms of lower variability among different samples of a pharmaceutical formulation. According to the present invention, the variability of dissolution is measured using USP apparatus 2, placing each of the 6 formulation examples in 900 ml 0.1 N HCL at 37 ±0.5 °C with paddle speed of 50 rpm after 10, 15, 20, 30 and 45 minutes. It is favorable that the RSD is below 20% for the first point and below 10% for the other points during dissolution test.

The term 'dissolution stability' as used herein refers to the similarity of dissolution profiles (similarity factor greater than 50, in comprasion to initial) obtained at different periods of storage at varying temperature and humidity conditions.

The term 'similarity factor' or 'f2 factor' as used herein refers to one way of comparing dissolution profiles of two different products. This model-independent mathematical approach compares the dissolution profile of the two products: test and reference or two strengths, or pre-and post-approved products from the same manufactures. Tests are recommended to be performed under the same test conditions. The dissolution time points for both the profiles should be the same, for example for immediate release products e.g.10, 15, 20, 30, 45 minutes. Only one time point should be considered after 85% dissolution of the reference product. An f2 value of 50 or greater (50-100) ensures sameness or equivalence of two curves, and thus performance of the two products. The similarity factor f2 should be computed using the equation:

$$f_2 = 50 * \log \frac{100}{\sqrt{1 + \frac{1}{n}\sum_{t=1}^{n}(R_t - T_t)^2}}$$

Where Rt and Tt are the cumulative percentage of the drug dissolved at each of the selected n time points of the comparison (reference) and (test) product respectively. For products which are very rapidly dissolving, i.e. more than 85% of the drug is dissolved within 15 minutes, dissolution profiles may be accepted as similar without further mathematical evaluation.

The pharmaceutical composition of the present invention generaly comprises solifenacin or a pharmaceutically acceptable salt thereof. The salts of solifenacin which can be used in the compositions of the present invention include the acid adduct salts with mineral acids such as hydrobromic acid, hydroiodic acid, sulfuric acid, nitric acid and phosphoric acid or organic acids such as formic, butyric acid, propionic acid, oxalic acid, malonic acid, fumaric acid, maleic acid,

lactic acid, malic acid, citric acid, tartaric acid, carbonic acid, metansulfonic acid, glutamic acid, and succinic acid, the latter being preferred. The composition preferably comprise solifenacin succinate salt.

The composition according to present invention includes a tablet, a capsule and the like. Preferably, the composition according to the present invention is in the form of a tablet.

In another aspect, the present invention discloses the preparation of a pharmaceutical composition, particularly tablets and capsules containing solifenacin succinate. Such a composition can be prepared with good content uniformity by a much simpler manufacturing wet granulation method utilizing simple equipment like a rapid mixer granulator.

In accordance with the above, the present invention discloses a method for the preparation of a tablet comprising solifenacin succinate, wherein the solifenacin is present substantially in amorphous form, and at least 2% w/w disintegrant, the method comprising

i. mixing solifenacin succinate at least with one pharmaceutically acceptable excipient such as diluents, disintegrants, binders, etc.
ii. granulating the mixture of step (i) with a binder solution and optionally a pharmaceutically acceptable excipient;
iii. drying the granules of step (ii);
iv. optionally compacting the step (iii) materials into compacts and subsequently milling and sizing through sieve;
v. optionally mixing the granules of step (iv) or step (iii) with at least one pharmaceutically acceptable excipient;
vi. compressing the granules of step (v) into a tablet.

The binder solution that was used in step (ii) for granulation can be prepared by dissolving a binder in a suitable solvent. The solvent can be organic solvent, water or mixtures thereof, preferably water. The amount of water that was used to make a binder solution can be in the range of adequate amount to make fine uniform granules.

The obtained granules were dried under vacuum at the temperature of about 50 to 60°C for about 3 to 4 hours. The obtained granules can be made of uniform size through sieving and optionally mixing the granules with one of the pharmaceutically acceptable excipents. The obtained dry uniform granules were compressed into a tablet.

Optionally the obtained tablets may be film coated in a conventional manner, e.g. for appearance, for palatability or for production purposes.

The term "disintegrant" as used in herein refers to any material that has wicking and /or swelling properties when it comes in contact with water. Suitable disintegrants include crospovidone, starch, pregelatinized starch, sodium starch glycolate, hydroxypropyl starch, microcrystalline cellulose, carboxymethylcellulose sodium or calcium, croscarmellose, polacrilin potassium, low-substituted hydroxypropylcellulose, sodium or calcium alginate, agar, guar gum, chitosan, alginic acid and mixtures thereof. Preferably, the disintegrant is pregelatinized starch. The composition preferably comprises 2 to 10 wt % of disintegrant, in particular 3 %.

A binder is a substance which holds the components of the composition together in the required composition form. Preferably the total weight of the composition is comprised of about 0.1 to about 30 wt % binder, more preferably about 1 to about 15 wt %, in particular about 1 to about 10 w/w % of a binders based on the total weight of the composition. Examples of binders which include but are not limited to, cellulose derivatives such as microcrystalline cellulose, hydroxymethyl cellulose, hydroxypropylcellulose.etc, sucrose" copovidone, and polyvinylpyrrolidone. Preferably, the binder is polyvinylpyrrolidone.

The term 'filler' and the term 'diluent' are herein used interchangeably. It is known that, in general, the term 'filler' is used in the context of capsular formulations and the term 'diluent' in tablet formulations. Fillers fill out the size of a composition, making it practical to produce and convenient for the consumer to use. Suitable filler/diluent include, but are not limited, to calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose lactose (e.g. spray-dried lactose, $\alpha$-lactose, $\beta$-lactose, Tabletose, various grades of Pharmatose®, Microtose® or Fast-Floc®), methylcellulose polymers such as, e.g., Methocel A, Methocel A4C, Methocel A4C, Methocel A 15C, Metocel A4M), hydroxyethylcellulose, hydroxypropylcellulose, L-hydroxypropylycellulose (low substituted), hydroxypropyl methylcellulose (HPMC) (e.g. Methocel E, F and K, Metolose SH of Shin-Etsu, grades of Methocel F and Metolose 65 SH, the 4,000, 15,000 and 100,000 cps grades of Methocel K; and the 4,000, 15,000, 39,000 and 100,000 grades of Metolose 90 SH), sodium carboxymethylcellulose, carboxymethylene, carboxymethylhydroxyethylcellulose and other cellulose derivatives, starches or modified starches (including potato starch, wheat starch, corn starch, rice starch, pregelatinised maize starch), magnesium carbonate, magnesium oxide, maltitol, maltodextrins, maltose, sorbitol, starch, sucrose, sugar, and xylitol, erytritol. More preferably diluent/filler is lactose. The amount of diluents used according to the present invention may be 20 to 95 w/w % , preferably about 50 to about 90 w/w% of a diluent, in particular about 60 to about 90 w/w% of a diluent, based on the total weight of the composition.

The presence of a lubricant is particularly preferred when the composition is a tablet as lubricants improve the tabletting process. Lubricants prevent composition ingredients from clumping together and from sticking to the tablet punches or capsule filling machine and improve flowability of the composition mixture. Suitable lubricants are preferably

selected from stearic acid, stearic acid salts such as magnesium stearate, palmitic acid salts such as magnesium palmitate, oleic acid salts such as magnesium oleate, sodium lauryl sulfate, magnesium lauryl sulfate, hydrogenated vegetable oil, hydrogenated castor oil, talc, sodium stearyl fumarate, macrogols or mixtures thereof. Preferably, the lubricant is sodium stearyl fumarate. The composition preferably comprises 0.5 to 10 wt % of lubricant.

In another aspect, the present invention discloses a stable formulation comprising amorphous solifenacin succinate free of stabilizers and antioxidants.

In another aspect, the present invention discloses s stable formulation comprising amorphous solifenacin succinate which is substantially free of crystalline solifenacin succinate, such that solifenacin succinate remains unchanged in amorphous form having longer shelf life period. Said composition is further substantially free of its degradation impurities.

In another aspect, the present invention discloses pharmaceutical formulations in the form of solid oral dosage forms which may be an immediate release form or a modified release form.

According to another aspect, the present invention discloses a solid pharmaceutical composition with improved storage stability comprising an amorphous form of solifenacin or pharmaceutically acceptable salts thereof, characterized in that it further comprises;

a) about 50 to about 90 wt% of a diluents,
b) about 2 to about 10 wt % of pregelatinized starch as a disintegrant
c) about 0.5 to about 10 wt % of a binder,
d) about 0.5 to about 10 wt % of a lubricant

each based on the total weight of the composition.

In another aspect, the present invention discloses a solid pharmaceutical composition comprising an amorphous form of solifenacin or pharmaceutically acceptable salts thereof, characterized in that it further comprises;

a) about 50 to about 90 wt% of lactose,
b) about 2 to about 10 wt % of pregelatinized starch as a disintegrant,
c) about 0.5 to about 10 wt % of a Polyvinypyrrolidone as a binder,
d) about 0.5 to about 10 wt % of a Sodium stearyl fumarate as a lubricant,

each baased on the total weight of the composition.

In another aspect, the present invention discloses a solid pharmaceutical composition comprising an amorphous form of solifenacin or pharmaceutically acceptable salts thereof, characterized in that it further comprises;

a) about 90 wt% of Lactose,
b) about 3 % of pregelatinized starch as a disintegrant,
c) about 1 % of a Polyvinypyrrolidone as a binder,
d) about 3 % of a Sodium stearyl fumarate as a lubricant,

each based on the total weight of the composition.

<u>**Example**</u>

Two pharmaceutical formulations with targeted amounts of 5 mg and 10 mg active ingredient with the following components were prepared.

| **CONTENT** | **5 mg Unit Formula** | **10 mg Unit Formula** |
|---|---|---|
| Solifenacin Succinate | 5.00 | 10.00 |
| Lactose | 136.50 | 131.50 |
| PVP K-30 | 1.50 | 1.50 |
| Pregelatinized Starch (Starch 1500) | 3.00 | 3.00 |
| Sodium Stearyl Fumarate | 4.00 | 4.00 |
| Deionized Water | q.s. | q.s. |
| **CORE TABLET** | *150.0* | *150.0* |

(continued)

| CONTENT | 5 mg Unit Formula | 10 mg Unit Formula |
|---|---|---|
| Opadry Yellow | 5.00 | - |
| Opadry Pink | - | 5.00 |
| Deionized Water | Sufficient amounts | Sufficient amounts |
| **FILM COATED TABLET** | *155.0* | *155.0* |
| *q.s. means a sufficient amount. | | |

The pharmaceutical compositions according to the present invention were manufactured according to processes known in the art.

The dissolution profile is obtained in accordance with pharmacopoeia requirements. Thus, the release of solifenacin succinate is tested by dissolution testing using Ph.Eur. apparatus 2 (paddle), rotation 50 rpm, 900 ml HCl 0.1 N and 37 °C measured on 6 tablets. The testing is carried out initially and after one month or three months in blister at 40 °C and 75% Relative Humidity (RH). The test is generally applicable in order to test whether the composition is stable with respect to dissolution profile.

Table 1. % Dissolved Solifenacin Succinate 5 mg Example Tablet

| Time, min. | initial | 1.month_40°C | 3.month_40°C |
|---|---|---|---|
| **10** | 67,6 | 57,7 | 63,7 |
| **15** | 90,1 | 88,3 | 92,7 |
| **20** | 95,9 | 95,1 | 94,6 |
| **30** | 96,3 | 96,9 | 96,0 |
| **45** | 95,4 | 96,9 | 95,9 |

Tablets of the present invention as well as of a commercial comprasion product were subjected to dissolution testing with attention to variability of dissolution. These results are shown in Table 2 and Table 3.

Table 2. Dissolution Profile of Commercial Comprasion Prodcut

| ***Sample*** | ***Commercial Comprasion Prodcut*** | | | | |
|---|---|---|---|---|---|
| | **10.min.** | **15.min.** | **20.min** | **30.min** | **45.min** |
| **1** | 25,4 | 48,6 | 66,0 | 81,1 | 91,7 |
| **2** | 54,4 | 79,5 | 94,6 | 103,6 | 104,3 |
| **3** | 30,5 | 49,1 | 61,2 | 77,0 | 90,0 |
| **4** | 53,2 | 78,6 | 93,2 | 102,0 | 102,8 |
| **5** | 53,5 | 80,6 | 96,7 | 104,2 | 104,5 |
| **6** | 52,5 | 72,9 | 92.6 | 99,5 | 101,5 |
| **RSD** | **29,51** | **22,33** | **19,01** | **12,90** | **6,59** |

Table 3. Dissolution Profile of Example

| ***Sample*** | ***Example*** | | | | |
|---|---|---|---|---|---|
| | **10.min.** | **15.min.** | **20.min** | **30. min** | **45.min** |
| **1** | 81,3 | 96,4 | 96,4 | 97,0 | 97,0 |
| **2** | 58,0 | 97,0 | 105,7 | 99,0 | 98,7 |

(continued)

| Sample | Example | | | | |
|---|---|---|---|---|---|
| | **10.min.** | **15.min.** | **20.min** | **30. min** | **45.min** |
| **3** | 63,5 | 97,8 | 100,3 | 98,8 | 98,9 |
| **4** | 71,0 | 98,0 | 99,1 | 98,3 | 98,2 |
| **5** | 74,8 | 96,7 | 97,3 | 98,0 | 97,9 |
| **6** | 59,1 | 90,9 | 98,9 | 100,4 | 100,0 |
| **RSD** | **13,67** | **2,74** | **3,30** | **1,15** | **1,03** |

## Claims

**1.** A solid pharmaceutical composition that has improved storage stability with respect to dissolution comprising at least 2% w/w disintegrant(s) and solifenacin or pharmaceutical acceptable salt thereof which is substantially free of crystalline form and free of degradation impurities, **characterized in that** the composition is prepared by wet granulation method.

**2.** The solid pharmaceutical composition according to claim1, wherein the disintegrant is pregelatinized starch.

**3.** The solid pharmaceutical composition according to claim1, which further comprise diluent, lubricant and binder.

**4.** The solid pharmaceutical composition according to claim 3, wherein the diluents are lactose, lactose monohydrate, microcrystalline cellulose, cellulose derivatives, starch, sucrose, mannitol, sorbitol.

**5.** The solid pharmaceutical composition according to claim(4), wherein the preffered diluent is lactose monohydrate.

**6.** The solid pharmaceutical composition according to claim 3, where in the lubricants are sodium stearyl fumarate, magnesium stearate and stearic acid.

**7.** The solid pharmaceutical composition according to claim 6, wherein the preferred lubricant is sodium stearyl fumarate

**8.** A solid pharmaceutical composition comprising an amorphous form of solifenacin or pharmaceutically acceptable salts thereof, **characterized in that** it further comprises;

a) about 50 to about 90 wt% of a diluents,
b) about 2 to about 10 wt % of pregelatinized starch as a disintegrant
c) about 0.5 to about 10 wt % of a binder,
d) about 0.5 to about 10 wt % of a lubricant

each based on the total weight of the composition.

**9.** A solid pharmaceutical composition according to claim 8, comprising an amorphous form of solifenacin or pharmaceutically acceptable salts thereof, **characterized in that** it further comprises;

a) about 50 to about 90 wt% of Lactose,
b) about 2 to about 10 wt % of pregelatinized starch
c) about 0.5 to about 10 wt % of a Polyvinypyrrolidone,
d) about 0.5 to about 10 wt % of a Sodium stearyl fumarate each baased on the total weight of the composition.

**10.** A solid pharmaceutical composition according to claim 9, comprising an amorphous form of solifenacin or pharmaceutically acceptable salts thereof, **characterized in that** it further comprises;

a) about 90 wt% of lactose,
b) about 3 % of pregelatinized starch

c) about 1 % of a polyvinypyrrolidone,
d) about 3 % of a sodium stearyl fumarate

each based on the total weight of the composition.

Europäisches Patentamt
European Patent Office
Office européen des brevets

# EUROPEAN SEARCH REPORT

Application Number
EP 13 00 1214

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 2 018 850 A1 (RATIOPHARM GMBH [DE]) 28 January 2009 (2009-01-28) * paragraph [0030]; claim 1 * | 1-10 | INV. C07D453/02 A61K31/439 A61K9/16 A61K47/08 A61K47/38 A61K47/30 |
| X,D | EP 2 415 472 A1 (ASTELLAS PHARMA INC [JP]) 8 February 2012 (2012-02-08) * paragraph [0012]; claims * | 1-10 | |
| X,D | EP 1 832 288 B1 (ASTELLAS PHARMA INC [JP]) 20 June 2012 (2012-06-20) * claims * | 1-10 | |
| X,D | WO 2008/128028 A2 (REDDYS LAB LTD DR [IN]; REDDYS LAB INC DR [US]; KHARWADE PRAMOD [IN];) 23 October 2008 (2008-10-23) * claims 1,2 * | 1-10 | |
| X | WO 2012/004264 A1 (RAGACTIVES S L U [ES]; FUENTES GERARDO GUTIERREZ [ES]; BONDE-LARSEN AN) 12 January 2012 (2012-01-12) * the whole document * | 1-10 | TECHNICAL FIELDS SEARCHED (IPC) |
| X | WO 2010/012459 A2 (KRKA D D NOVO MESTO [SI]; RUZIC MILOS [SI]; PRUDIC DARJA [SI]; PECAVAR) 4 February 2010 (2010-02-04) * claim 2; examples 14-22 * | 1-10 | C07D A61K |
| X | WO 2008/013851 A2 (TEVA PHARMA [IL]; TEVA PHARMA [US]; KOLTAI TAMAS [IL]; NIDAM TAMAR [IL) 31 January 2008 (2008-01-31) * paragraphs [0128], [0137], [0144] * | 1-10 | |
| A | WO 2008/011462 A2 (REDDYS LAB LTD DR [IN]; REDDYS LAB INC DR [US]; MATHAD VIJAYAVITTHAL T) 24 January 2008 (2008-01-24) * claims 1-4 * | 1-10 | |

-/--

The present search report has been drawn up for all claims

2

| Place of search | Date of completion of the search | Examiner |
|---|---|---|
| Munich | 15 April 2013 | Giese, Hans-Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 00 1214

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2009/087664 A1 (CADILA HEALTHCARE LTD [IN]; DAVE MAYANK GHANSHYAMBHAI [IN]; PANDEY BIP) 16 July 2009 (2009-07-16) * claims * ----- | 1-10 | |
| | | | TECHNICAL FIELDS SEARCHED (IPC) |

The present search report has been drawn up for all claims

2

| Place of search | Date of completion of the search | Examiner |
|---|---|---|
| Munich | 15 April 2013 | Giese, Hans-Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 00 1214

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2013

| Patent document cited in search report | | Publication date | Patent family member(s) | | | Publication date |
|---|---|---|---|---|---|---|
| EP 2018850 | A1 | 28-01-2009 | CA | 2693179 | A1 | 29-01-2009 |
| | | | EA | 201000039 | A1 | 30-08-2010 |
| | | | EP | 2018850 | A1 | 28-01-2009 |
| | | | EP | 2178507 | A1 | 28-04-2010 |
| | | | WO | 2009012987 | A1 | 29-01-2009 |
| EP 2415472 | A1 | 08-02-2012 | EP | 2415472 | A1 | 08-02-2012 |
| | | | JP | 4816828 | B2 | 16-11-2011 |
| | | | US | 2010273825 | A1 | 28-10-2010 |
| | | | WO | 2010113840 | A1 | 07-10-2010 |
| EP 1832288 | B1 | 20-06-2012 | AU | 2005320672 | A1 | 06-07-2006 |
| | | | CA | 2591761 | A1 | 06-07-2006 |
| | | | CN | 101141961 | A | 12-03-2008 |
| | | | EP | 1832288 | A1 | 12-09-2007 |
| | | | KR | 20070098889 | A | 05-10-2007 |
| | | | US | 2008103171 | A1 | 01-05-2008 |
| | | | WO | 2006070735 | A1 | 06-07-2006 |
| WO 2008128028 | A2 | 23-10-2008 | EP | 2146693 | A2 | 27-01-2010 |
| | | | WO | 2008128028 | A2 | 23-10-2008 |
| WO 2012004264 | A1 | 12-01-2012 | CA | 2804107 | A1 | 12-01-2012 |
| | | | EP | 2590973 | A1 | 15-05-2013 |
| | | | WO | 2012004264 | A1 | 12-01-2012 |
| WO 2010012459 | A2 | 04-02-2010 | EP | 2310387 | A2 | 20-04-2011 |
| | | | WO | 2010012459 | A2 | 04-02-2010 |
| WO 2008013851 | A2 | 31-01-2008 | EP | 2043639 | A2 | 08-04-2009 |
| | | | US | 2008114028 | A1 | 15-05-2008 |
| | | | WO | 2008013851 | A2 | 31-01-2008 |
| WO 2008011462 | A2 | 24-01-2008 | EP | 2046751 | A2 | 15-04-2009 |
| | | | US | 2009326230 | A1 | 31-12-2009 |
| | | | WO | 2008011462 | A2 | 24-01-2008 |
| WO 2009087664 | A1 | 16-07-2009 | EP | 2229387 | A1 | 22-09-2010 |
| | | | EP | 2484681 | A1 | 08-08-2012 |
| | | | EP | 2489666 | A2 | 22-08-2012 |
| | | | JP | 2011505416 | A | 24-02-2011 |
| | | | US | 2010298371 | A1 | 25-11-2010 |
| | | | WO | 2009087664 | A1 | 16-07-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0801067 A
- EP 1728791 A1
- EP 2018850 A1
- EP 2415472 A1
- EP 2400954 A1
- EP 1832288 B1
- WO 2008128028 A2